(19) Europäisches Patentamt
European Patent Office
Office européen des brevets

(11) **EP 3 398 913 A1**

(12) **EUROPÄISCHE PATENTANMELDUNG**

(43) Veröffentlichungstag:
    **07.11.2018 Patentblatt 2018/45**

(51) Int Cl.:
    **C02F 11/04** (2006.01)        **C12M 1/107** (2006.01)
    **C12M 1/00** (2006.01)         C02F 3/28 (2006.01)
    C02F 1/36 (2006.01)         C02F 1/44 (2006.01)
    C02F 3/34 (2006.01)         C02F 1/78 (2006.01)
    C02F 1/38 (2006.01)

(21) Anmeldenummer: 18170857.9

(22) Anmeldetag: **04.05.2018**

(84) Benannte Vertragsstaaten:
    **AL AT BE BG CH CY CZ DE DK EE ES FI FR GB**
    **GR HR HU IE IS IT LI LT LU LV MC MK MT NL NO**
    **PL PT RO RS SE SI SK SM TR**
    Benannte Erstreckungsstaaten:
    **BA ME**
    Benannte Validierungsstaaten:
    **KH MA MD TN**

(30) Priorität: **05.05.2017 EP 17169714**

(71) Anmelder: **Hochschule für Angewandte**
    **Wissenschaften Hof**
    **95028 Hof (DE)**

(72) Erfinder: **Schmid, Andreas**
    **95703 Schönkirch (DE)**

(74) Vertreter: **Engelhard, Markus**
    **Boehmert & Boehmert**
    **Anwaltspartnerschaft mbB**
    **Pettenkoferstrasse 22**
    **80336 München (DE)**

(54) **VERFAHREN UND VORRICHTUNG ZUR LEISTUNGSSTEIGERUNG ANAEROBER ABBAUVERFAHREN DURCH ERWEITERUNG BZW. ANPASSUNG DER VORVERSÄUERUNGSSTUFE**

(57)    Die vorliegende Erfindung bezieht sich auf eine Vorrichtung zur Vorversäuerung, ein Verfahren zur Vorversäuerung und die Verwendungen der Vorrichtung und des Verfahrens, bei der Herstellung von Biogas oder anderen anaeroben Abbauprodukten auf Basis organischer Ausgangssubstrate oder in der Abwasser- und Schlammbehandlung.

**Figur 3**

**Beschreibung**

Die vorliegende Erfindung bezieht sich auf eine Vorrichtung zur Vorversäuerung, ein Verfahren zur Vorversäuerung und die Verwendungen der Vorrichtung und des Verfahrens, bei der Herstellung von Biogas oder anderen anaeroben Abbauprodukten auf Basis organischer Ausgangssubstrate oder in der Abwasser- und Schlammbehandlung.

**Hintergrund der Erfindung**

Anaerobe Reaktoren, insbesondere zur anaeroben Abwasserreinigung, lassen sich nach der Art des Biomasserückhalts und nach der Art der Biomasse unterscheiden. Diese kann flockig oder granulär vorliegen oder auf Trägermaterialien angesiedelt sein. Weiterhin lassen sich anaerobe Reaktoren nach der Raumabbauleistung unterscheiden, wobei derzeit die EGSB (expanded granular sludge blanket)- und Fließbettreaktoren die höchsten Raumabbauleistungen auch im praktischen Betrieb nachgewiesen haben [1, 5]. In Deutschland wurden bis zum Dezember 2008 ca. 250 großtechnische Anaerobanlagen zur Industrieabwasserbehandlung errichtet [6, 7]. Wichtige Kenngrößen für die Reaktorauslegung sind das Schlammalter und die Schlammbelastung, da nicht der Reaktor, sondern die im Reaktor befindliche Biomasse für die Abbauleistung verantwortlich ist. Der Biomassengehalt ist wiederum eine Funktion des Biomassenrückhalts im System, so dass zur Vereinfachung der Betrachtung die CSB (Chemischer Sauerstoffbedarf)-Raumbelastung herangezogen wird. Wesentlicher Bestandteil der anaeroben Anlagen ist die Hydrolyse-Stufe (auch Vorversäuerung genannt), die auch dann wichtig ist, wenn bereits ein hoher Gehalt an flüchtigen Säuren im Abwasser vorliegt. Diese Stufe kann getrennt angelegt sein (ggf. auch in Kombination mit einem hydraulischen Puffer) oder - abhängig vom gewählten Verfahren - in den Anaerobreaktor integriert werden. Derzeit erfolgt eine pH-Anpassung und gegebenenfalls Stickstoff- und Phosphordosierung nach dem Vorversäuerungsreaktor unmittelbar im Zustrom des Anaerobreaktors [1].

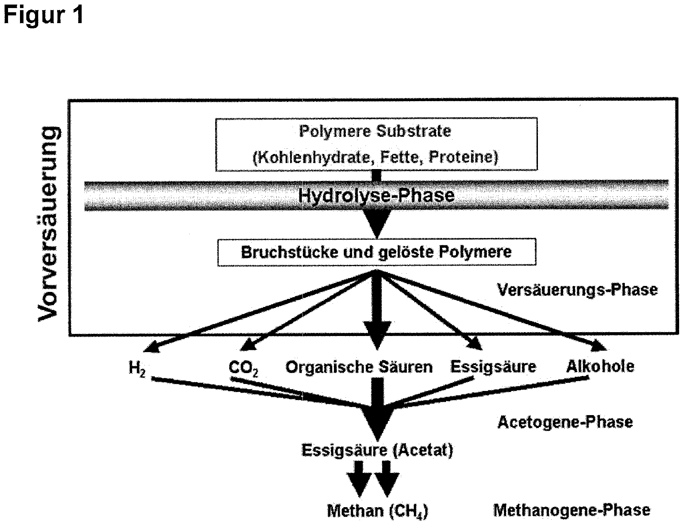

Zum Ausgleich von Mengen-, Konzentrations- und Frachtschwankungen haben sich Misch- und Ausgleichsbecken bewährt. Diese Anlagen können gleichzeitig zur gezielten Versäuerung genutzt werden, wobei die Prozesse des Ausgleichs und der Versäuerung schwer zu entkoppeln sind. Durch die vorgeschaltete Vorversäuerung wird der 4-stufige anaerobe Abbau auf zwei Reaktoren aufgeteilt (siehe Figur 1), was i.d.R. die nachfolgende Aceto- und Methanogenese stabilisiert und die Gefahr einer Übersäuerung drastisch vermindert [1].

Abhängig von der Abwasserart werden in der industriellen Abwasserreinigung für Ausgleich und Versäuerung Aufenthaltszeiten von 6 bis 24 Stunden gewählt. Bei Untersuchungen von Weiland und Wulfert [5, 6] mit Kartoffelschlempe kam es bei Überschreitung der Verweilzeit von 24 Stunden zur Bildung von Buttersäure, Valeriansäure und Capronsäure. Bei Versuchen mit anderen agrarindustriellen Abläufen nahm darüber hinaus die Konzentration von Propionsäure zu. Bei den meisten Untersuchungen zeigte sich, dass eine Verweilzeit von ca. 24 Stunden aus technisch-ökonomischen Gründen das Optimum darstellt. Einige Abwässer (zum Beispiel Kaffeeproduktionsabwasser) weisen allerdings auch bei niedrigeren Aufenthaltszeiten von 6 h noch einen hohen Versäuerungsgrad von 80 % auf [7].

Der optimale Versäuerungsgrad (Verhältnis von CSB der organischen Säuren zum filtrierten CSB) ist system- und abwasserspezifisch. Von Pelletschlammreaktoren ist bekannt, dass der Versäuerungsgrad Einfluss auf die Pelletstruktur hat. Ein vollständig versäuertes Abwasser führt nach [8] zum Zerfall der Pellets. Eine stabile Versäuerung von Schlempe aus der Zitronensäureproduktion erreichte Moser [9] bei Aufenthaltszeiten von ca. 2-3 Tagen durch Rückführung von Schlamm aus dem Methanreaktor (EKJ-Reaktor) in den Versäuerungsreaktor.

Derzeit finden sich Vorversäuerungssysteme vorwiegend bei anaeroben Hochschlastreaktoren und werden in der kommunalen Schlammbehandlung als auch in der landwirtschaftlichen Biogastechnik nicht angewendet [10].

WO 2009/155587 A2 beschreibt eine Vorrichtung beziehungsweise ein Verfahren zur Erzeugung von Methangas, bei dem mit Hilfe einer Anode, Kathode und methanogenen Mikroorganismen ausgestatteten Reaktors Methan erzeugt wird. Eine Vorversäuerung etc. wird nicht beschrieben.

EP 2628788 A1 beschreibt ein Verfahren und eine Anlage zur Herstellung eines Methanisierungssubstrats aus faseriger Biomasse. Dabei wird die Biomasse verdünnt und zerkleinert.

EP 1559688 A1 beschreibt ein Verfahren zum Abbau von organischem Schlamm, bei dem der Schlamm nacheinander einer anaeroben und aeroben Fermentation unterzogen wird.

Die Gewinnung von Biogas aus nachwachsenden Rohstoffen mit anschließender Verstromung wird im Erneuerbare Energie Gesetz (EEG) 2014 stark eingeschränkt. Die Folgen für die Biogasbranche sind dramatisch. Beim Anlagenneubau droht ein völliges Aus, bei den bestehenden Anlagen zumindest ein weitgehender Innovationsstopp. Die über Jahrzehnte gerade auch in Bayern erarbeitete Kompetenz in diesem Bereich droht mit den entsprechenden Firmen verloren zu gehen. Landwirten, Genossenschaften und Kommunen, die sich im Bereich Biogas engagiert haben, fehlt die Perspektive. Dabei könnte Biogas, als originär stofflicher Energieträger, wichtige Funktionen im Kontext der Energiewende erfüllen (Treibstoff, Energiespeicher, Regelenergieträger).

Dazu müsste in diesem Bereich allerdings ein verfahrens- und prozesstechnisches Umdenken stattfinden. Die Anlagen der Zukunft müssen deutlich "intelligenter" und vor allem flexibler werden, sowohl was ihre "Produkte" und deren Verwendung als auch was ihre "Rohstoffbasis" betrifft. Gleichzeitig muss die Biogasproduktion in ein Gesamtkonzept Abfall/Energie integriert werden. Während dies ab einer gewissen Anlagengröße (> 30'000 t/a) bereits machbar ist, gibt es für kleine (landwirtschaftliche) bis mittlere (kommunale) Anlagen außerhalb der Großstädte, bislang keine wirtschaftlich tragbaren technischen Lösungen.

Es besteht ein Bedarf im Stand der Technik nach verbesserten Mitteln und Methoden in der Schlammbehandlung und landwirtschaftlichen Biogasnutzung.

*Vorrichtung zur Vorversäuerung*

Die Aufgabe wird erfindungsgemäß durch die Bereitstellung einer Vorrichtung zur Vorversäuerung gelöst.

In einem Aspekt betrifft die Erfindung eine Vorrichtung zur Vorversäuerung von organischem Substrat, insbesondere zur Verwendung im Rahmen eines nachgeschalteten anaeroben Abbauverfahrens, wobei die Vorrichtung umfasst:

(a) einen Reaktor ("Vorversäuerungsreaktor"), umfassend

(i) ein vorgeschaltetes Zerkleinerungsaggregat, für organisches Substrat und/oder Cosubstrat(e),
(ii) einen Zulauf für die Zuführung von Substrat und/oder Cosubstrat, bevorzugt besagtem organischen Substrat und/oder Cosubstrat;
(iii) jeweils einen Abfluss und Zufluss zum Verbinden des Reaktors im Umlauf mit mindestens einem Desintegrations-System;
(iv) einen Ablauf, bevorzugt zum Verbinden des Reaktors mit einem anaeroben Reaktor;
(v) Rührmittel, wie Rührwerk oder einer internen oder externen Umwälzpumpe;

wobei die Vorrichtung weiterhin umfasst:

(b) mindestens einen Desintegrations-System, das sich mit dem Reaktor (a) in Umlaufverbindung befindet; und/oder

(c) einen internen Schlammkreislauf für die Anreicherung von hydrolytischen und acidophilen Mikroorganismen; und/oder

(d) eine Zugabevorrichtung zur Dosierung von Phosphor und/oder Stickstoff in den Reaktor. In einer Ausführungsform ist der interne Schlammkreislauf ein sich innerhalb des Reaktors befindender Schlammkreislauf. In einer Ausführungsform umfasst die Vorrichtung außer dem Reaktor a) jedes von b) und d).

Der Begriff "Desintegration", wie hierin verwendet, bezieht sich auf einen Aufschluss von Zellen durch Aufbrechen von Zellwänden und Zellmembranen. Bei einem solchen Aufschluss werden Zellinhaltsstoffe freigesetzt und bevorzugt in Lösung gebracht, die Zellen werden nicht jedoch notwendigerweise zerkleinert. Der Begriff "Desintegration", wie hierin verwendet, kann auch synonym mit dem Begriff "Aufschluss" verwendet werden. Demzufolge kann ein "Desintegrations-System" auch als "Aufschluss-System" bezeichnet werden.

In einer Ausführungsform umfasst die Vorrichtung zwei oder mehrere Desintegrations-Systeme (b), und/oder wobei das oder die Desintegrations-Systeme (b) Mittel zur Durchführung eines Kavitationsverfahrens, Mittel zur Durchführung einer Elektroporation, eine Rührwerkskugelmühle, einen Scherspalthomogenisator, Mittel zur Durchführung eines Ultraschallverfahrens, Mittel zur Durchführung eines Elektroimpulsverfahrens, eine Lysat-Zentrifuge, Mittel zur Enzymzugabe, Mittel zur Durchführung von Autolyse, Mittel zur Durchführung von Nassoxidation, Mittel zur Ozondosierung, Mittel zur Durchführung von alkalischer Hydrolyse oder Mittel zur Durchführung von saurer Hydrolyse, oder Kombinationen davon umfasst (umfassen).

In einer Ausführungsform umfasst die Vorrichtung zwei Desintegrations-Systeme, die Mittel zur Durchführung eines Kavitationsverfahrens und Mittel zur Durchführung einer Elektroporation umfassen oder darstellen. In einer Ausführungsform kann ein Desintegrations-System gemäß der vorliegenden Erfindung auch so ausgestaltet sein, dass es sowohl Mittel zur Durchführung eines Kavitationsverfahrens als auch Mittel zur Durchführung einer Elektroporation umfasst oder enthält. Es ist weiterhin bevorzugt, dass das Kavitationsverfahren ein hydrodynamisches Kavitationsverfahren ist.

In einer Ausführungsform steht (stehen) das oder die Desintegrations-Systeme (b) mit dem Reaktor (a) im Bypass-Rücklaufstrom in Umlaufverbindung. Der Begriff "Bypass-Rücklaufstrom", wie hierin verwendet, bedeutet im Zusammenhang mit dem oder den Desintegrations-Systemen, dass das Desintegrations-System oder die Desintegrations-Systeme sich außerhalb des Reaktors und mit diesem in Umlaufverbindung befinden. Der Reaktorinhalt oder Teile davon können durch das oder die Desintegrations-Systeme laufen (und desintegriert werden) und anschließend dem Reaktor wieder zugeführt werden.

In einer Ausführungsform umfasst der interne Schlammkreislauf (c):
Mittel zur Sedimentation im Reaktor, wie etwa einen Schrägklärer vor dem Ablauf, eine separate Sedimentationsstufe, und/oder Mittel zur internen und/oder externen Filtration.

Der Begriff "interner Kreislauf" oder "interner Schlammkreislauf", wie hierin verwendet, bezeichnet bevorzugt die Möglichkeit des Rückhalts und Anreicherung von Mikroorganismen und Feststoffen im Reaktor. Die hierfür erforderlichen Mittel können sich innerhalb des Reaktors, aber auch außerhalb davon befinden. Beispielsweise kann der im Reaktor befindliche Schlamm oder die im Rektor befindliche Biomasse aus dem Reaktor verbracht und extern gefiltert und anschließend das Filtrat in den Reaktor zurückgeführt werden. Auch dies wird durch den Begriff "interner Kreislauf" oder "interne Rezirkulation" umfasst.

In einer Ausführungsform umfasst die Vorrichtung weiterhin:

- mindestens ein Rückhaltesystem für die Anreicherung von hydrolytischen und/oder acidophilen Mikroorganismen und/oder für die Anreicherung von Feststoffen, zum Beispiel eine Abscheideeinrichtung oder Abscheidemittel zum Zurückhalten von Feststoffen und/oder von hydrolytischen und acidophilen Mikroorganismen, und/oder von Feststoffen, insbesondere ein (oder mehrere) Rückhaltesystem(e) ausgewählt aus:

  Mitteln zur Filtration innerhalb oder außerhalb des Reaktors, insbesondere Mittel zur internen oder externen Crossflow-Filtration, beispielsweise Mikro- oder Ultrafiltration, wobei, bevorzugt, die Mittel zur Filtration in Nassaufstellung oder Trockenaufstellung vorgesehen sind; Mittel zur Feststoffrückhaltung, insbesondere mittels Zentrifugation; Mittel zur Sedimentation in einem dem Reaktor nachgeschalteten Becken, beispielsweise einem Zwischenklärbecken; Mittel zur Sedimentation im Reaktor, bevorzugt in einer oder mehreren strömungsberuhigten Zonen des Reaktors; und Kombinationen davon;
  und/oder Mittel zur pH-Wert-Anpassung im Reaktor (a) oder im Ablauf des Reaktors.

*Verfahren zur Vorbehandlung*

In einem weiteren Aspekt betrifft die Erfindung ein Verfahren zur Vorbehandlung, insbesondere Vorversäuerung, von organischem Substrat, umfassend die Schritte:

- Bereitstellen eines zu behandelnden organischen Substrats in einer Vorversäuerungsvorrichtung umfassend einen Reaktor, insbesondere einer Vorrichtung gemäß der vorliegenden Erfindung,
- Durchführen einer Hydrolyse und Acidogenese des organischen Substrats mit Hilfe von hydrolytischen und/oder acidophilen Mikroorganismen,

wobei im Rahmen der Hydrolyse/Acidogenese des organischen Substrats

  (i) mindestens ein im Umlauf mit dem Reaktor verbundenes/betriebenes Desintegrations-Verfahren durchgeführt; und/oder
  (ii) ein innerhalb des Reaktors befindlicher Schlammkreislauf für hydrolytische und/oder acidophile Mikroorganismen betrieben; und/oder
  (iii) Stickstoff und/oder Phosphor, nach Bedarf der Mikroorganismen, in den Reaktor eindosiert; und/oder
  (iv) optional, eine pH-Wert-Anpassung durchgeführt

wird.

In einer Ausführungsform werden zwei oder mehrere Desintegrations-Verfahren (i) durchgeführt, und/oder wobei das oder die Desintegrations-Verfahren (i) Kavitationsverfahren, Elektroporation, Mahlen mit einer Rührwerkskugelmühle, Homogenisieren mittels eines Scherspalthomogenisators, Ultraschallverfahren, Elektroimpulsverfahren, Zentrifugation mittels einer Lysatzentrifuge, Aufschluss mittels Enzymzugabe, Autolyse, Nassoxidation, Ozondosierung, alkalische Hydrolyse, saure Hydrolyse, und/oder Kombinationen davon umfasst (umfassen). In einer Ausführungsform ist das Kavitationsverfahren ein hydrodynamisches Kavitationsverfahren.

In einer Ausführungsform werden zwei Desintegrationsverfahren (i) durchgeführt wobei diese Desintegrationsverfahren bevorzugt ein Kavitationsverfahren und eine Elektroporation sind. Insbesondere ist dabei bevorzugt, dass das Kavitationsverfahren ein hydrodynamisches Kavitationsverfahren ist.

In einer Ausführungsform werden die Desintegrationsverfahren für eine Dauer im Bereich von 10 min bis 5 h, bevorzugt von 30 min bis 3 h, bevorzugter von 30 min bis 2 h durchgeführt.

In einer Ausführungsform ist das erfindungsgemäße Verfahren zur Vorbehandlung, insbesondere Vorversäuerung, von organischem Substrat, einem Verfahren zum anaeroben Abbau von organischem Substrat vorgeschaltet. In einer Ausführungsform ist das erfindungsgemäße Verfahren zur Vorbehandlung, insbesondere Vorversäuerung, von organischem Substrat einem Verfahren zum aeroben Abbau von organischem Substrat weder vorgeschaltet noch nachgeschaltet.

In einer Ausführungsform umfasst das Verfahren weiterhin eine Anreicherung der hydrolytischen und/oder acidophilen Mikroorganismen, bevorzugt durch Sedimentation und/oder Filtration, insbesondere während oder nach der Hydrolyse des organischen Substrats.

In einer Ausführungsform umfasst das Verfahren weiterhin

eine Vorbehandlung des organischen Substrats und/oder von Co-Substraten, beispielsweise Biomüll oder nachwachsenden Rohstoffe,

bevorzugt umfassend eine mechanische Zerkleinerung des organischen Substrats und/oder der Co-Substrate.

In einer Ausführungsform findet bei den Verfahren der vorliegenden Erfindung vor der Hydrolyse keine Verdünnung des Substrats zu oder in einer Flüssigkeit statt.

In einer Ausführungsform umfasst das Verfahren weiterhin das Zurückhalten von Feststoffen, optional kombiniert mit einem Zurückhalten von hydrolytischen und/oder acidophilen Mikroorganismen zum Aufbau eines internen Schlammkreislaufs.

*Verwendung*

In einem weiteren Verfahren betrifft die Erfindung eine Verwendung der Vorrichtung und/oder des Verfahrens gemäß der vorliegenden Erfindung,

- in der Biogasherstellung, insbesondere der landwirtschaftlichen Biogasherstellung,
- zur Klärschlammbehandlung,
- in der industriellen Abwasserreinigung,
- in der kommunalen Abwasserbehandlung,
- in der kommunalen Schlammbehandlung,
- zur Behandlung von organischen Abfällen,
- zur Behandlung von nachwachsenden Rohstoffen,
- zur Behandlung von Schlämmen und/oder Abwässern aus der Nahrungsmittelindustrie, chemischen Industrie, oder pharmazeutischen Industrie, und/oder
- zur kombinierten Behandlung oben genannter Einsatzstoffe, insbesondere von Klärschlamm, industriellem Abwasser, kommunalem Abwasser, kommunalem Schlamm, organischen Abfällen, nachwachsenden Rohstoffen, Schlämmen und/oder Abwässern aus der Nahrungsmittelindustrie, chemischen Industrie und/oder pharmazeutischen Industrie, als Co-Substratbeigaben.

*Verfahren zum anaeroben Abbau*

In einem weiteren Aspekt betrifft die vorliegende Erfindung ein Verfahren zum anaeroben Abbau von organischem Substrat, umfassend:

a) Durchführung eines Verfahrens zur Vorversäuerung gemäß der vorliegenden Erfindung, bevorzugt in einer Vorrichtung gemäß der vorliegenden Erfindung, mit einem organischen Substrat;

b) Anaerober Abbau des (der) aus (a) resultierenden Produkts (Produkte) des Vorversäuerungsverfahrens zu diversen organischen Zwischen- und Endprodukten wie z.B. Acetat, Formiat, Propionat, Butyrat, Valerianat, Ethanol, Propanol, Butanol, Wasserstoff, weiteren organischen Säuren oder Alkoholen, und/oder Methan, bevorzugt mittels Acetogenese und/oder Methanisierung,

wobei a) räumlich und/oder zeitlich getrennt von b) durchgeführt wird.

In einer Ausführungsform umfasst das Verfahren zum anaeroben Abbau von organischem Substrat gemäß der vorliegenden Erfindung keinen aeroben Abbau des aus dem Verfahren zur Vorversäuerung gemäß der vorliegenden Erfindung resultierenden Produkts. In einer Ausführungsform schließt daher ein Verfahren zum anaeroben Abbau von organischem Substrat gemäß der vorliegenden Erfindung einen aeroben Abbau aus.

Der Begriff "Vorrichtung zur Vorversäuerung", wie hierin verwendet, bezeichnet eine Kombination eines Reaktors mit mindestens einem Desintegrations-System und einem internen Schlammkreislauf. Der Begriff "Reaktor", wie hierin verwendet, bezeichnet einen Reaktor innerhalb einer erfindungsgemäßen Vorrichtung zur Vorversäuerung ("Vorversäuerungsvorrichtung"); er kann deshalb auch als "Vorversäuerungsreaktor" im engeren Sinne bezeichnet werden. Bei Einsatz einer erfindungsgemäßen Vorversäuerungsvorrichtung bzw. eines erfindungsgemäßen Vorversäuerungsreaktors im Rahmen eines anaeroben Abbauverfahrens, findet in der Vorversäuerungsvorrichtung bzw. dem Vorversäuerungsreaktor eine Hydrolyse und Versäuerung statt, in deren Rahmen aus organischen Substraten/Co-Substraten, bspw. organischen polymeren Substraten zunächst hydrolysierte Bruchstücke und gelöste organische Verbindungen und hieraus dann kurzkettige organische Säuren, Alkohole, $CO_2$ und $H_2$ entstehen. Die im Rahmen eines anaeroben Abbauverfahrens sich anschließende Bildung von Essigsäure, bzw. Methan (in der so genannten acetogenen Phase, bzw. methanogenen Phase) findet nicht in der erfindungsgemäßen Vorversäuerungsvorrichtung bzw. dem erfindungsgemäßen Vorversäuerungsreaktor statt. Hierdurch wird eine zeitliche und/oder räumliche Trennung der Vorversäuerungsphase, bestehend aus Hydrolyse und Versäuerung von der sich anschließenden Essigsäure/Methanproduktion erzielt. Die in der erfindungsgemäßen Vorversäuerungsvorrichtung durchgeführte Vorversäuerung kann optimiert und entsprechend den jeweiligen Bedürfnissen angepasst werden. In einem Vorversäuerungsreaktor bzw. einer Vorversäuerungsvorrichtung gemäß der vorliegenden Erfindung findet deshalb bevorzugt ausschließlich eine Hydrolyse und/oder Versäuerung, jedoch keine gezielte acetogene Phase und/oder methanogene Phase ("Methanisierung") statt. Der Begriff "Anaerobreaktor", oder anaerober Reaktor, wie hierin verwendet, bezeichnet einen Reaktor, in dem bevorzugt eine acetogene Phase bzw. eine methanogene Phase stattfindet, nicht jedoch eine Hydrolyse und/oder Versäuerung. Die beiden Vorgänge "Hydrolyse" und "Versäuerung", wie hierin verwendet, können auch gemeinsam als "Vorversäuerungsphase" bezeichnet werden. Der Begriff "intern" und "extern", wie hierin verwendet, bspw. im Kontext eines Reaktors oder einer Vorrichtung, bspw. einer Vorversäuerungsvorrichtung, bezieht sich darauf, dass der genannte Bestandteil sich innerhalb (oder außerhalb) des Reaktors bzw. der Vorrichtung befindet, oder aber die Möglichkeit vorgesehen ist, Schlamm bzw. Biomasse bzw. Mikroorganismen in die Vorrichtung zurückzuführen. Bspw. befindet sich ein "interner Schlammkreislauf" als Bestandteil einer Vorrichtung in der Regel innerhalb dieser Vorrichtung. Ein solcher "interner Schlammkreislauf" kann sich jedoch auch innerhalb des Reaktors, der ja Bestandteil der Vorrichtung ist, befinden.

Beispielsweise kann auch ein außerhalb des Reaktors befindlicher externer Filter Teil des internen Schlammkreislaufs sein, sofern gewährleistet ist, dass das Filtrat in den Reaktor zurückgeführt werden kann.

In einer Vorversäuerungsvorrichtung gemäß der vorliegenden Erfindung, insbesondere innerhalb eines Vorversäuerungsreaktors gemäß der vorliegenden Erfindung kann ein Rückhaltesystem oder können mehrere Rückhaltesysteme vorgesehen sein. Ein solches Rückhaltesystem kann für die Anreicherung von hydrolytischen und/oder acidophilen Mikroorganismen zum Aufbau eines internen Schlammkreislaufs eingesetzt werden. Ein solches Rückhaltesystem kann auch oder alleine für die Anreicherung von Feststoffen vorgesehen sein. In einer Ausführungsform ist ein Rückhaltesystem vorgesehen, das sowohl für die Anreicherung von hydrolytischen und acidophilen Mikroorganismen als auch für die Anreicherung von Feststoffen vorgesehen ist. In einer anderen Ausführungsform der vorliegenden Erfindung ist ein Rückhaltesystem ausschließlich für die Anreicherung von hydrolytischen und/oder acidophilen Mikroorganismen vorgesehen und ein weiteres Rückhaltesystem für die Anreicherung von Feststoffen. Ein Beispiel für ein erfindungsgemäßes Rückhaltesystem ist eine Abscheideeinrichtung oder ein Abscheidemittel zum Abscheiden bzw. Zurückhalten von Feststoffen und/oder hydrolytischen und/oder acidophilen Mikroorganismen. Weitere Beispiele von Rückhaltesystemen in Übereinstimmung mit Ausführungsformen der vorliegenden Erfindung sind Mittel zur Filtration innerhalb oder außerhalb des Reaktors oder Vorrichtung, insbesondere Mittel zur internen oder externen Cross-Flow-Filtration. Beispiele hierfür sind die Mikrofiltration oder die Ultrafiltration. Die Mittel zur Filtration können in Nassaufstellung oder Trockenaufstellung vorgesehen sein. Weitere Beispiele für Rückhaltesysteme gemäß der vorliegenden Erfindung sind Mittel zur Feststoffrückhaltung mittels Zentrifugation. Ein typisches Beispiel hierfür ist eine Zentrifuge, die Bestandteil der Vorrichtung sein kann, oder bei Bedarf hinzugezogen werden kann. Eine entsprechende Zentrifuge kann auch in die Vorrichtung integriert sein. Weitere Beispiele für Rückhaltesysteme gemäß der vorliegenden Erfindung sind Mittel zur Sedimentation in einem nachgeschalteten Becken. Ein solches Becken kann dem Reaktor gemäß der vorliegenden Erfindung nachgeschaltet sein. Ein solches Becken kann beispielsweise ein Zwischenklärbecken sein, welches nach Durchführung der Vorversäuerung dazu dient, Feststoffe und/oder Mikroorganismen zu sedimentieren und den geklärten Überstand dann anschließend zur weiteren Verarbeitung im Rahmen des weiteren anaeroben Abbaus (bspw. im Rahmen einer Acetogenese und/oder Methanogenese) in einem separaten Reaktor, dem Anaerobenreaktor, verfügbar zu machen. Weitere Beispiele für Rückhaltesystem gemäß der vorliegenden Erfindung sind beispielsweise Mittel zur Sedimentation innerhalb des Reaktors in strömungsberuhigten Zonen und/oder in eingebauten Parallelplattenabscheider / Schrägklärer. Der Begriff "Biomasse" oder "Substrat", wie hierin verwendet, bezeichnet jegliche organische Masse aus tierischen und/oder pflanzlichen Quellen. In einer Ausführungsform bezeichnet der Begriff pflanzliche Bestandteile, tierische Bestandteile, pflanzliche Abfälle, tierische Abfälle, Abwasser, nachwachsende Rohstoffe, kommunale Schlämme, industrielle Schlämme und/oder Gülle. Ein Substrat, das gemäß der vorliegenden Erfindung verarbeitet werden kann, ist bevorzugt einer oder mehrere der vorgenannten Bestandteile. In einer bevorzugten Ausführungsform sind die im Rahmen der vorliegenden Erfindung verwendeten Biomassenbestandteile Substrate insbesondere pflanzlichen Ursprungs. Beispiel für pflanzliche Substrate sind Holz, Naturfasern, Pflanzenöle, Zucker und Stärke aus Pflanzen, usw. Von ebenfalls besonderer Bedeutung für den anaeroben Abbau sind tierische Abfälle, insbesondere Gülle. Der Begriff "Biomasse" wird hierin manchmal auch als "Substrat" oder "organisches Substrat" bezeichnet. Ein solches Substrat oder organisches Substrat ist typischerweise biologisch abbaubar. Organisches Substrat, welches biologisch abbaubar ist, ist Ausgangspunkt der vorliegenden Erfindung und wird mittels der erfindungsgemäßen Vorrichtung gemäß dem erfindergemäßen Verfahren behandelt, insbesondere vorbehandelt, d.h. vorversäuert. In einer Ausführungsform schließt der Begriff "Substrat" oder "Biomasse" solche Substrate aus, die bereits vorversäuert sind oder sein können, bspw. Silage, ohne dass sie mit einem erfindungsgemäßen Verfahren vorbehandelt worden wären.

Ebenfalls von Bedeutung sind kommunale oder industrielle Abwässer, organische Abfälle, insbesondere Biomüll aus Haushalten und Klärschlämme aus Kommune und Industrie. Die genannten Bestandteile können auch als "Cosubstrate" eingesetzt werden, in der Gestalt, dass in der Vorrichtung gemäß der vorliegenden Erfindung neben einem Hauptbestandteil (Substrat), beispielsweise Pflanzenabfall, auch ein weiterer Bestandteil (als Cosubstrat) eingesetzt wird, beispielsweise Biomüll.

*Weitere Beschreibung von Ausführungsformen des erfindungsgemäßen Verfahrens und der erfindungsgemäßen Vorrichtung*

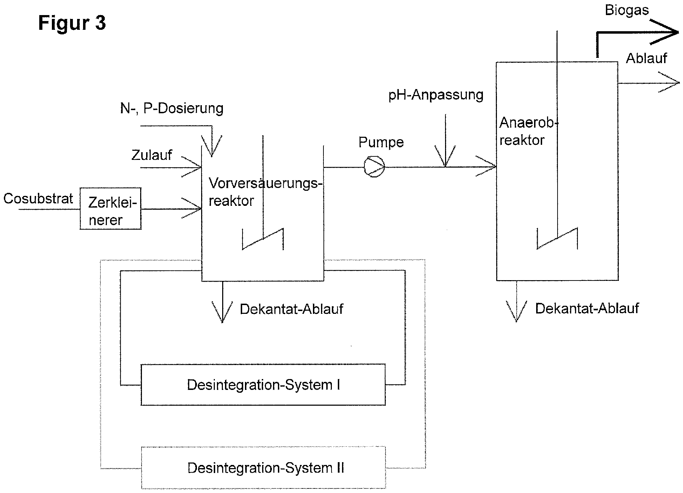

Figur 3 zeigt eine *Ausführungsform einer* erfindungsgemäßen Vorrichtung zur Vorversäuerung und das entsprechende Verfahren.

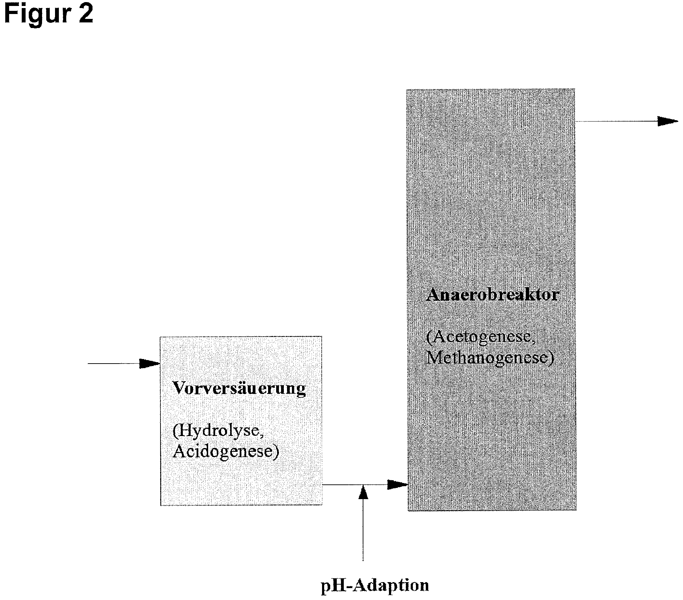

Zur Verbesserung der Betriebsstabilität wird vor dem eigentlichen Anaerobreaktor ein Vorversäuerungsreaktor vorgeschaltet (siehe Figur 2), was die Gefahr einer möglichen Übersäuerung im Anaerobreaktor durch eine pH-Wert-Adaption drastisch vermindert. Diese Betriebsweise wird derzeit nur in der industriellen Abwasserreinigung verwendet.

Für die Verwendung alternativer Cosubstrate (z.B. Biomüll) können diese Stoffe vor Eintrag in den Vorversäuerungsreaktor mechanisch zerkleinert werden. Etwaig eingetragene Feststoffe sollen durch eine Abscheideeinrichtung im Vorversäuerungsreaktor zurück gehalten werden (z.B. Einbau eines Parallelplattenabscheiders).

Durch großzügige Dimensionierung wird in einer Ausführungsform ein eigener Schlammkreislauf der hydrolytischen und acidogenen Mikroorganismen aufgebaut, was die Effizienz zusätzlich begünstigt. Ein solcher interner Schlammkreislauf wird derzeit technisch nicht in anderen Verfahren/Vorrichtungen zur Vorversäuerung angewendet.

Darüber hinaus erlaubt in einer Ausführungsform eine Dosierung von Stickstoff und Phosphor mittels einer Zugabevorrichtung die Adaption eines optimalen C:N:P-Verhältnisses zur Leistungssteigerung der hydrolytischen und acidophilen Mikroorganismen im Vorversäuerungsreaktor. Eine solche Dosierung wird derzeit technisch nicht in anderen Verfahren/Vorrichtungen zur Vorversäuerung angewendet.

Um die Hydrolyse dieser alternativen Substrate als auch die der verwendeten Schlämme zu beschleunigen, werden in einer Ausführungsform ein oder verschiedene/mehrere Desintegrationsverfahren in der Vorversäuerung im Bypass - Rücklaufstrom/Umlaufstrom betrieben; was derzeit im Stand der Technik in anderen Verfahren/Vorrichtungen nicht angewendet wird.

Die vorgenannten Ausführungsformen können alternativ oder in beliebigen Kombinationen vorgesehen sein. In einer bevorzugten Ausführungsform sind sowohl Schlammkreislauf als auch Desintegrationssystem als auch Zugabevorrichtung in einer erfindungsgemäßen Vorrichtung vorgesehen. Im erfindungsgemäßen Verfahren können diese wiederum getrennt als Alternative oder aber in beliebigen Kombinationen betrieben werden.

Bei Verwendung von z.B. Biomüll oder nachwachsenden Rohstoffen sollte dieser bevorzugt vor Eintrag in den Vorversäuerungsreaktor zerkleinert werden, um die Desintegrationssysteme nicht zu beeinträchtigen und die hydrolytischen Vorgänge zu beschleunigen.

Wegen der säuretoleranten Eigenschaften der hydrolytischen und acidophilen Mikroorganismen kann die pH-Anpassung sowohl im Vorversäuerungsreaktor als auch im Zustrom zum Anaerobreaktor erfolgen. Diese pH-Adaption verringert in erheblichem Maße die Gefahr einer Übersäuerung im Anaerobreaktor und führt zu einer weiteren Stabilisierung des Gesamtprozesses.

*Effekte durch das neue Verfahren bzw. die neue Vorrichtung:*

Durch die Dosierung von Stickstoff und Phosphor in den Vorversäuerungsreaktor werden die hydrolytischen und acidogenen Mikroorganismen optimal mit Nährstoffen versorgt. Eine Wachstumslimitierung dieser Organismen kann somit deutlich vermindert werden, was die mikrobiellen Vorgänge signifikant beschleunigt. Dies ermöglicht hohe Umsatzraten und kleine Reaktorvolumina für den Vorversäuerungsreaktor. Die Verweildauer des Substrats in der Vorversäuerungsvorrichtung bzw. dem Vorversäuerungsreaktor und die Vorversäuerung insgesamt kann von 6 - 24 h auf ca. 1 - 4 h verringert werden.

Eine solche Stickstoff und Phosphor -Dosierung erfolgt derzeit nicht in kommerziell erhältliche Reaktoren - dies geht deutlich über den derzeitigen Stand der Technik hinaus.

Der interne Schlammkreislauf durch z.B. Sedimentation der Mikroorganismen und gegebenenfalls Rückführung in den Vorversäuerungsreaktor führt zu höheren Biomassenkonzentrationen und einer Anreicherung von speziell angepassten Mikroorganismen im System. Dies beschleunigt zusätzlich die hydrolytischen und acidogenen Vorgänge, was zu geringen Reaktorvolumina und höheren Umsatzraten führt. Der Biomassenrückhalt kann z.B. durch Sedimentation im eigentlichen Reaktor erfolgen (Einbau von Schrägklärern vor dem Ablauf oder im ganzen Reaktor durch Ausschalten des Rührwerks in Analogie zu sequencing batch reactors - SBR-Verfahren) oder durch eine separate Sedimentationsstufe (in Analogie zu Belebungsverfahren) als auch durch interne und externe Filtration (in Analogie zu Membranverfahren in der Abwasserreinigung).

Interne Schlammkreisläufe für einen Vorversäuerungsreaktor existieren derzeit nach dem Stand der Technik nicht; auch eine Implementierung der Schlammrückführung geht deutlich über den derzeitigen Stand der Technik hinaus.

Die im Umlauf betriebenen Desintegrationsverfahren (z.B. Kavitationsverfahren, Elektroporation, etc.) unterstützen die Hydrolyse, insbesondere wenn organische Feststoffe zugeführt werden (Cosubstrate - z.B. Biomüll oder nachwachsende Rohstoffe).

Darüber hinaus unterstützen diese Desintegrationsverfahren die Freisetzung von Zellinhaltsstoffen bei z.B. der Schlammbehandlung (Schlammfaulung auf Kläranlagen). Damit wird ein hoher Aufschlussgrad unterschiedlicher Rohsubstrate erreicht und vor dem/für den nachfolgenden Anaerobreaktor (Acetogenese & Methanogenese - siehe Figur 1) relativ konstante Bedingungen eingestellt. Damit kann der eigentliche Anaerobreaktor deutlich unabhängiger von den eingesetzten Substraten betrieben werden, was zu einer signifikanten Steigerung der Betriebsstabilität führt.

Der durch die Desintegration herbeigeführte höhere Aufschlussgrad der Rohsubstrate (mikrobiell leicht verfügbar) ermöglicht anschließend im Anaerobreaktor höhere Mengen an erzeugtem Biogas oder anderen anaeroben Zwischenprodukten, was die Leistungsfähigkeit des Anaerobreaktors deutlich steigert - damit sind langfristig kleinere Anaerobreaktoren möglich.

Die im Bypass des Vorversäuerungsreaktors im Rücklaufstrom betriebenen Desintegrationsverfahren erlauben durch Änderung der Betriebszeit/Volumenströme und Variation der eingetragenen Leistung eine Anpassung des Aufschlussgrades in Abhängigkeit der eingesetzten Rohsubstrate. Damit kann unmittelbar auf die Qualität und Quantität der zuströmenden Rohsubstrate reagiert werden, um einen optimalen Aufschlussgrad für die nachfolgende Anaerobstufe zu erreichen.

Desintegrationsverfahren werden derzeit nach dem Stand der Technik nicht im Vorversäuerungsreaktor verwendet; eine Implementierung von Desintegrationsverfahren geht deutlich über den derzeitigen Stand der Technik hinaus.

*Anwendungspotential des neuen Verfahrens*

Durch das vorgestellt Verfahren können nunmehr anaerobe Fermentationsanlagen nahezu unabhängig vom Substrat (z.B. nachwachsende Rohstoffe, Schlämme, organische Abfälle) betrieben werden, was den oben genannten Wirtschaftszweigen, insbesondere kleinen (landwirtschaftlichen) bis mittleren (kommunalen) Anlagen außerhalb der Großstädte, eine erweiterte Wachstumsperspektive bietet.

Die vorliegende Erfindung wird in den folgenden Figuren und dem Beispiel weiter verdeutlicht, ohne jedoch darauf beschränkt zu sein. Die zitierten Referenzen sind hiermit durch Bezugnahme vollständig aufgenommen.

**Figuren**

**Figur 1.** Separation des anaeroben Abbaus durch Vorversäuerung.

**Figur 2.** Zweistufige Betriebsweise durch vorgeschaltete Vorversäuerung.

**Figur 3.** Verfahrensfließbild der erweiterten Vorversäuerungsstufe.

**Figur 4.** Schema einer erfindungsgemäßen Substratvorbehandlungsanlage zusammen mit einem anaeroben Reaktor.

**Figur 5.** Verringerung der Viskosität durch Elektroporation im Vergleich zur Kavitation.

Beispiel

Zusammenfassung des Beispiels:

Mittels eines 800 Liter Vorversäuerungsreaktors, aufgebaut in einem Klärwerk, wurde eingedickter Überschussschlamm durch Elektroporation und hydrodynamische Kavitation im Batchbetrieb bis zu 6 Stunden lang behandelt. Faulschlamm aus dem Anaerobreaktor des Klärwerks wirkte stabilisierend und unterstützend auf die Vorversäuerung.

Zudem wurden Versuche mit Biomüll und Silage aus Silphie als Cosubstrat durchgeführt. Bei der anschließenden Fermentation in Laborreaktoren zeigten sich für Elektroporation 19 %, hydrodynamische Kavitation 21 % und in Kombination beider Desintegrationsverfahren bis zu 31 % höhere Gaserträge. Zudem verbesserten sich die rheologischen Eigenschaften des behandelten Substrates.

**a) Aufbau der Substratvorbehandlungsanlage**

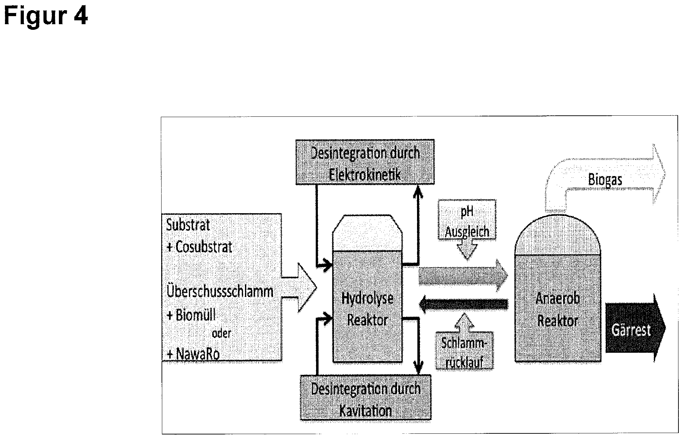

Die Versuchsanlage besteht aus einem Vorversäuerungsreaktor mit einem Volumen von 800 Litern. Das Substrat wird auf halber Reaktorhöhe abgepumpt, durchläuft eine Behandlung durch zwei Elektroporationselektroden Typ Bio-TURBO-Crack® der Firma INNOVUM GmbH mit einer Leistung je Modul von 35 Watt und einer Hochspannung von 100 kV und eine Kavitationsdüse mit anschließender 1 m langer Kavitationsstrecke. Zum Vergleich kann die Anlage mit keiner oder nur einer Desintegrationsmethode betrieben werden. Das Hydrolysat wurde in 2 Liter Anaerobreaktoren fermentiert (Figur 4). Durch Batchbetrieb des Hydrolyserealctors wurde Überschussschlamm bis zu 6 Stunden offen vorversäuert. Eine Schlammrückführung aus dem Anaerobreaktor des Klärwerks sorgte für eine ausreichende Anzahl an hydrolytischen und acidogenen Bakterien im Reaktor. Die Menge betrug dabei 1,3 % des Gesamtsubstratvolumens im Vorversäuerungsreaktor.

Zusätzlich wurde das Substrat durch Elektroporation und hydrodynamische Kavitation vorbehandelt. Das vorversäuerte Substrat wurde nach pH-Ausgleich in Laborreaktoren anaerob abgebaut und die Biogasausbeute ermittelt. Typische Verweilzeiten in der Schlammfaulung betragen 20 Tage. Die Bioreaktoren wurden im Verhältnis 1:1 mit Hydrolysat und Faulschlamm gefüllt. Für die Bewertung wurde die Ausbeute an Normbiogas des vorbehandelten Substrates in der ersten Fermentationswoche mit der des unbehandelten Substrates verglichen und der Aufschlussgrad ACSB der Desintegrationsmethoden bestimmt. Durch 22 stündigen Aufschluss einer 1:1 mit 1 molarer Natronlauge versetzten Schlammprobe wird die maximale Freisetzung an CSB erreicht. Die Proben werden 10 Minuten bei 10 000 g zentrifugiert, über eine Membran (0,45 $\mu$m) gefiltert und abschließend der CSB der Probe gemessen. Der Aufschlussgrad $A_{CSB}$ lässt sich über Gleichung 1 (s.u.) bestimmen. $CSB_{desint}$ ist der Sauerstoffbedarf im Schlammwasser des desintegrierten Schlammes in [mg/l]. $CSB_u$ der Sauerstoffbedarf im Schlammwasser des unbehandelten Schlammes in [mg/l] und $CSB_{NaOH}$ der Sauerstoffbedarf im Schlammwasser des Referenzaufschlusses in [mg/l].

$$A_{CSB} = \left[ \frac{CSB_{desint} - CSB_u}{CSB_{NaOH} - CSB_u} \right] \cdot 100[\%] \qquad (Gl.\ 1)$$

Als Cosubstrate wurden 10 % Volumenanteil an Biomüll oder Silage aus *Silphie* zugesetzt.

**b) Steigerung der Biogasausbeute und verbesserte Viskosität durch die Vorbehandlung**

Durch die Desintegrationsmethoden wurde ein nahezu linearer Anstieg des Aufschluss-grades im Desintegrat abhängig von der Behandlungszeit erreicht. Allerdings ergab ein hoher Aufschlussgrad nicht zwangsläufig eine hohe Gasausbeute. Nach 4 stündiger Behandlung ergaben sich Aufschlussgrade von 3 - 6 % mit Biomüll als Co-substrat sogar bis 12 %.

Die optimalen Behandlungszeiten, laut den Ergebnissen der Gasausbeute, liegen allerdings bei 30 Minuten bis 2 Stunden. In Tabelle 1 wird der Anstieg der Biogasausbeute durch eine Vorversäuerung gegenüber dem unbehandelten Substrat angegeben. Energie wurde nur durch eine Pumpe zum Umwälzen eingetragen. Es zeigten sich durch eine Stunde Behandlung gute Verbesserungen im Gasertrag von 12 bis 14 % und 8 % beim der Vorversäuerung von Biomüll als Cosubstrat. Bei Silage war die Verbesserung der Gasausbeute geringer.

## Tabelle 1: Steigerung der Biogasausbeute durch Vorversäuerung

| Substrat / Parameter | Dickschlamm | Dickschlamm + 10% Biomüll | Dickschlamm + 10% Silage |
|---|---|---|---|
| Optimale Behandlungszeit | 1 Std. | 1 Std. | 2 Std. |
| Energieaufwand | 2,1 kWh/m³ | 2,1 kWh/m³ | 4,2 kWh/m³ |
| Anstieg Gasertrag | + 12 – 14 % | + 8 % | + 1 % |

## Tabelle 2: Steigerung der Biogasausbeute durch Vorversäuerung und Desintegration mit Elektroporation

| Substrat / Parameter | Dickschlamm | Dickschlamm + 10% Biomüll | Dickschlamm + 10% Silage |
|---|---|---|---|
| Optimale Behandlungszeit | 1 Std. | 1 Std. | 30 min. |
| Energieaufwand | 2,2 kWh/m³ | 2,2 kWh/m³ | 1,1 kWh/m³ |
| Anstieg Gasertrag | + 10 % | + 8 – 18 % | + 4 % |

Durch hydrodynamische Kavitation konnte bei einer Behandlungsdauer von 3 Stunden eine Steigerung des Gasertrages um 21 % erreicht werden. Allerdings war aufgrund eines zu geringen Düsendurchmessers keine Behandlung der Cosubstrate mit Kavitation möglich. Eine kombinierte Behandlung von Dickschlamm durch Elektroporation und Kavitation im Reaktor zeigte eine Steigerung von bis zu 31 %.

## Tabelle 3: Steigerung der Biogasausbeute durch Vorversäuerung mit hydrodynamischer Kavitation und hydrodynamischer Kavitation kombiniert mit Elektroporation

| Substrat / Parameter | Dickschlamm mit Kavitation | Dickschlamm mit Kavitation und Elektroporation | Cosubstrat Biomüll/Silage |
|---|---|---|---|
| Optimale Behandlungszeit | 3 Std. | 30 min. - 2 Std. | -* |
| Energieaufwand | 3,1 kWh/m³ | 1,6 – 6,5 kWh/m³ | - |
| Anstieg Gasertrag | + 21 % | + 13 – 31 % | - |

* Partikel der Cosubstrat führten zur Verblockung der Kavitationsdüse

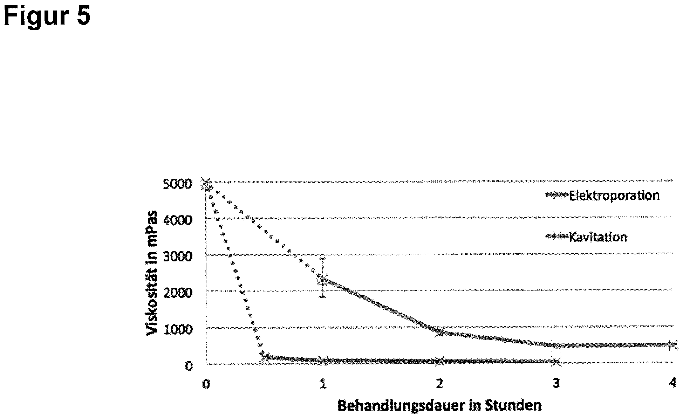

Während der Versuche zeigte sich, dass sich die Viskosität des Überschuss-schlammes durch Elektroporation deutlich verringern lässt (Figur 5). Schon nach kurzen Behandlungszeiten von 15 Minuten ließ sich eine deutliche Verringerung der Viskosität von über 4000 mPas auf ca. 200 mPas feststellen. Mit Kavitation war eine deutlich längere Behandlungsdauer nötig, um die Viskosität signifikant zu verringern.
Roher Schlamm weist in der Regel Werte von ca. 5000 mPas oder höher auf. Die Viskosität zum Startzeitpunkt konnte nicht ermittelt werden, da sich mit dem verwendeten Kugelfallviskosimeter erst ab 4000 mPas die Viskosität ermitteln lässt. Partikelgrößenmessungen zeigten, dass sich größere Schlammflocken um bis zu 15 % verkleinerten, während sich bei mittleren und kleinen Partikeln kaum eine Veränderung zeigte. Messungen mit Biomüll oder Silage im Desintegrat konnten nicht durchgeführt werden, da sich zu große Partikel für die verwendeten Methoden in den Proben vorfanden.

**c) Zusammenfassung und Ausblick**

Die durchgeführten Versuche zeigen, dass der Einsatz von Vorversäuerungsreaktoren zu einer verbesserten Gasausbeute aus Überschussschlamm führt. Bei Substraten, bei denen durch ihre Lagerung schon eine Säurebildung eintritt, wie z.B. bei Silage, ist eine zusätzliche Vorversäuerung nur in Verbindung mit geeigneter Desintegration zielführend. Zudem wird die Pumpfähigkeit des Substrates durch eine Vorbehandlung verbessert. Kombinierte Substratvorbehandlung durch Elektroporation und Kavitation erreicht eine Verbesserung der Gasausbeute um bis zu 31 %.

**Referenzen**

[1] Bischofsberger, W.; Dichtl, N.; Rosenwinkel, K.-H.; Seyfried, C.F.; Böhnke, B.: Anaerobtechnik. 2. Auflage, Springer Verlag Berlin Heidelberg, 2005.

[2] H. Meyer: Leistungsfähigkeit anaerober Reaktoren zur Industrieabwasserreinigung, Veröffentlichungen des Instituts für Siedlungswasserwirtschaft und Abfalltechnik der Universität Hannover, Heft 128, 2004.

[3] Mitglieder der DWA-Arbeitsgruppe IG-5.1: Auswahl und Bewertung von Systemen und Reaktoren zur anaeroben Industrieabwasserbehandlung. Korrespondenz Abwasser, 59 (1), 36-44, 2012.

[4] Mitglieder der DWA-Arbeitsgruppe IG-5.1: Anaerobe Reaktoren und ihre Einsatzbereiche. Korrespondenz Abwasser, 56 (11), 1147 -1152, 2009.

[5] Weiland, P., Wulfert, K.: Fixed Bed Reactors for Anaerobic Treatment of High Strength Wastewaters - Development and Application, BTF - BIOTECH-FORUM, Heft 3, S. 151-158, 1986.

[6] Weiland, P.; Wulfert, K.: Entsorgung einer Bioethanol-Demonstrationsanlage, Chem. Ind., 97-99, 1986.

[7] Alexiou, I. E. Anderson, G. K., Evison, L. M.: Design of pre-acidification reactors for the anaerobic treatment of industrial wastewaters, Water Science & Technology, 29 (9), 199-204, 1994.

[8] Lettinga, G., Hulshoff Pol, L. W.: UASB-process design for various types of wastewaters, Water Science & Technology, 24 (8), 87-107, 1991.

[9] Moser, D.: Anaerobe Abwasserreinigung - Beeinflussende Faktoren der Versäuerung eines Zitronensäurefabrikabwassers, Wiener Mitteilungen Wasser - Abwasser - Gewässer, Band 173, 2002.

[10] Mitglieder der DWA-Arbeitsgruppe IG-5.1: Auswahl und Bewertung von Systemen und Reaktoren zur anaeroben Industrieabwasserbehandlung. Korrespondenz Abwasser, 59 (1), 36-44,2012.

**Patentansprüche**

1.  Verfahren zur Vorbehandlung, insbesondere Vorversäuerung, von organischem Substrat, umfassend die Schritte:

    - Bereitstellen eines zu behandelnden organischen Substrats in einer Vorversäuerungsvorrichtung umfassend

        (a) einen Reaktor ("Vorversäuerungsreaktor"), umfassend

            (i) ein vorgeschaltetes Zerkleinerungsaggregat, für organisches Substrat und/oder Cosubstrat(e),
            (ii) einen Zulauf für die Zuführung von Substrat und/oder Cosubstrat;

(iii) jeweils einen Abfluss und Zufluss zum Verbinden des Reaktors im Umlauf mit mindestens einem Desintegrations-System;

(iv) einen Ablauf, bevorzugt zum Verbinden des Reaktors mit einem anaeroben Reaktor;

(v) Rührmittel, wie Rührwerk oder einer internen oder externen Umwälzpumpe;

wobei die Vorrichtung weiterhin umfasst:

(b) mindestens einen Desintegrations-System, das sich mit dem Reaktor (a) in Umlaufverbindung befindet; und/oder

(c) einen internen Schlammkreislauf für die Anreicherung von hydrolytischen und acidophilen Mikroorganismen; und/oder

(d) eine Zugabevorrichtung zur Dosierung von Phosphor und/oder Stickstoff in den Reaktor.

- Durchführen einer Hydrolyse und Acidogenese des organischen Substrats mit Hilfe von hydrolytischen und/oder acidophilen Mikroorganismen,

wobei im Rahmen der Hydrolyse/Acidogenese des organischen Substrats

(i) mindestens ein im Umlauf mit dem Reaktor verbundenes/betriebenes Desintegrations-Verfahren durchgeführt; und/oder

(ii) ein innerhalb des Reaktors befindlicher Schlammkreislauf für hydrolytische und/oder acidophile Mikroorganismen betrieben; und/oder

(iii) Stickstoff und/oder Phosphor, nach Bedarf der Mikroorganismen, in den Reaktor eindosiert; und/oder

(iv) optional, eine pH-Wert-Anpassung durchgeführt

wird.

2. Verfahren nach Anspruch 1, wobei zwei oder mehrere Desintegrations-Verfahren (i) durchgeführt werden, und/oder wobei das oder die Desintegrations-Verfahren (i) Kavitationsverfahren, Elektroporation, Mahlen mit einer Rührwerkskugelmühle, Homogenisieren mittels eines Scherspalthomogenisators, Ultraschallverfahren, Elektroimpulsverfahren, Zentrifugation mittels einer Lysatzentrifuge, Aufschluss mittels Enzymzugabe, Autolyse, Nassoxidation, Ozondosierung, alkalische Hydrolyse, saure Hydrolyse, und/oder Kombinationen davon umfasst (umfassen).

3. Verfahren nach Anspruch 1 oder 2, wobei zwei Desintegrationsverfahren (i) durchgeführt werden, die ein Kavitationsverfahren und eine Elektroporation sind.

4. Verfahren nach einem der vorangehenden Ansprüche, weiterhin umfassend eine Anreicherung der hydrolytischen und/oder acidophilen Mikroorganismen, bevorzugt durch Sedimentation und/oder Filtration, insbesondere während oder nach der Hydrolyse des biologischen Substrats.

5. Verfahren nach einem der vorangehenden Ansprüche, weiterhin umfassend

eine Vorbehandlung des organischen Substrats und/oder von Co-Substraten, beispielsweise Biomüll oder nachwachsenden Rohstoffe,

bevorzugt umfassend eine mechanische Zerkleinerung des organischen Substrats und/oder der Co-Substrate.

6. Verfahren nach einem der vorangehenden Ansprüche, weiterhin umfassend das Zurückhalten von Feststoffen, optional kombiniert mit einem Zurückhalten von hydrolytischen und/oder acidophilen Mikroorganismen zum Aufbau eines internen Schlammkreislaufs.

7. Vorrichtung zur Vorversäuerung von organischem Substrat, insbesondere zur Verwendung im Rahmen eines nachgeschalteten anaeroben Abbauverfahrens, wobei die Vorrichtung umfasst:

(a) einen Reaktor ("Vorversäuerungsreaktor"), umfassend

(i) ein vorgeschaltetes Zerkleinerungsaggregat, für organisches Substrat und/oder Cosubstrat(e),

(ii) einen Zulauf für die Zuführung von Substrat und/oder Cosubstrat;

(iii) eine Zugabevorrichtung zur Dosierung von Phosphor und/oder Stickstoff;

(iv) jeweils einen Abfluss und Zufluss zum Verbinden des Reaktors im Umlauf mit mindestens einem Desintegrations-System;

(v) einen Ablauf, bevorzugt zum Verbinden des Reaktors mit einem anaeroben Rektor,

(vi) Rührmittel, wie Rührwerk oder einer internen oder externen Umwälzpumpe;

(b) mindestens einen Desintegrations-System, das sich mit dem Reaktor (a) in Umlaufverbindung befindet,
(c) einen internen Schlammkreislauf für die Anreicherung von hydrolytischen und acidophilen Mikroorganismen.

**8.** Vorrichtung gemäß Anspruch 7, umfassend zwei oder mehrere Desintegrations-Systeme (b),
und/oder wobei das oder die Desintegrations-Systeme (b) Mittel zur Durchführung eines Kavitationsverfahrens, Mittel zur Durchführung einer Elektroporation, eine Rührwerkskugelmühle, einen Scherspalthomogenisator, Mittel zur Durchführung eines Ultraschallverfahrens, Mittel zur Durchführung eines Elektroimpulsverfahrens, eine Lysat-Zentrifuge, Mittel zur Enzymzugabe, Mittel zur Durchführung von Autolyse, Mittel zur Durchführung von Nassoxidation, Mittel zur Ozondosierung, Mittel zur Durchführung von alkalischer Hydrolyse oder Mittel zur Durchführung von saurer Hydrolyse, oder Kombinationen davon umfasst (umfassen).

**9.** Vorrichtung nach einem der Ansprüche 7 oder 8, wobei das oder die Desintegrations-Systeme (b) mit dem Reaktor (a) im Bypass-Rücklaufstrom in Umlaufverbindung stehen.

**10.** Vorrichtung gemäß einem der Ansprüche 7 bis 9, wobei der interne Schlammkreislauf (c) umfasst:
Mittel zur Sedimentation im Reaktor, wie etwa einen Schrägklärer vor dem Ablauf, eine separate Sedimentationsstufe, und/oder Mittel zur internen und/oder externen Filtration.

**11.** Vorrichtung gemäß einem der Ansprüche 7 bis 10, weiterhin umfassend:

- mindestens ein Rückhaltesystem für die Anreicherung von hydrolytischen und/oder acedophilen Mikroorganismen und/oder für die Anreicherung von Feststoffen, zum Beispiel eine Abscheideeinrichtung oder Abscheidemittel zum Zurückhalten von Feststoffen und/oder von hydrolytischen und acidophilen Mikroorganismen, und/oder von Feststoffen, insbesondere ein (oder mehrere) Rückhaltesystem(e) ausgewählt aus:

Mitteln zur Filtration innerhalb oder außerhalb des Reaktors, insbesondere Mittel zur internen oder externen Crossflow-Filtration, beispielsweise Mikro- oder Ultrafiltration, wobei, bevorzugt, die Mittel zur Filtration in Nassaufstellung oder Trockenaufstellung vorgesehen sind; Mittel zur Feststoffrückhaltung, insbesondere mittels Zentrifugation; Mittel zur Sedimentation in einem dem Reaktor nachgeschalteten Becken, beispielsweise einem Zwischenklärbecken; Mittel zur Sedimentation im Reaktor, bevorzugt in einer oder mehreren strömungsberuhigten Zonen des Reaktors; und Kombinationen davon;
und/oder Mittel zur pH-Wert-Anpassung im Reaktor (a) oder im Ablauf des Reaktors.

**12.** Verwendung des Verfahrens nach einem der Ansprüche 1 bis 6 und/oder des Verfahrens nach einem der Ansprüche 7-11,

- in der Biogasherstellung, insbesondere der landwirtschaftlichen Biogasherstellung,
- zur Klärschlammbehandlung,
- in der industriellen Abwasserreinigung,
- in der kommunalen Abwasserbehandlung,
- in der kommunalen Schlammbehandlung,
- zur Behandlung von organischen Abfällen,
- zur Behandlung von nachwachsenden Rohstoffen,
- zur Behandlung von Schlämmen und/oder Abwässern aus der Nahrungsmittelindustrie, chemischen Industrie, oder pharmazeutischen Industrie, und/oder
- zur kombinierten Behandlung oben genannter Einsatzstoffe, insbesondere von Klärschlamm, industriellem Abwasser, kommunalem Abwasser, kommunalem Schlamm, organischen Abfällen, nachwachsenden Rohstoffen, Schlämmen und/oder Abwässern aus der Nahrungsmittelindustrie, chemischen Industrie und/oder pharmazeutischen Industrie, als Co-Substratbeigaben.

**13.** Verfahren zum anaeroben Abbau von organischem Substrat, umfassend:

a) Durchführung eines Verfahrens zur Vorversäuerung gemäß einem der Ansprüche 1-6, bevorzugt in einer Vorrichtung gemäß einem der Ansprüche 7-11, mit einem biologischen Substrat;
b) Anaerober Abbau des (der) aus (a) resultierenden Produkts (Produkte) des Vorversäuerungsverfahrens zu organischen Zwischen- und Endprodukten,

wie z.B. Acetat, Formiat, Propionat, Butyrat, Valerianat, Ethanol, Propanol, Butanol, Wasserstoff, weiteren organischen Säuren oder Alkoholen, und/oder Methan, bevorzugt mittels Acetogenese und/oder Methanisierung,

wobei a) räumlich und/oder zeitlich von b) durchgeführt wird.

# Figur 1

# Figur 2

**Vorversäuerung**

(Hydrolyse,
Acidogenese)

**Anaerobreaktor**

(Acetogenese,
Methanogenese)

pH-Adaption

**Figur 3**

N-, P-Dosierung

Zulauf

Cosubstrat | Zerklei-nerer

Vorversäuerungs-reaktor

Pumpe

pH-Anpassung

Anaerob-reaktor

Biogas

Ablauf

Dekantat-Ablauf

Dekantat-Ablauf

Desintegration-System I

Desintegration-System II

# Figur 4

# Figur 5

**Europäisches Patentamt**
**European Patent Office**
**Office européen des brevets**

# EUROPÄISCHER RECHERCHENBERICHT

Nummer der Anmeldung

EP 18 17 0857

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 628 788 A1 (METHANEO [FR]) 21. August 2013 (2013-08-21) * Abbildung 1 * * Absätze [0020] - [0067] * * Absatz [0071] * * Absatz [0078] * * Absätze [0001] - [0011] * ----- | 1-13 | INV. C02F11/04 C12M1/107 C12M1/00 ADD. C02F3/28 C02F1/36 |
| X | EP 1 559 688 A1 (SEGHERS KEPPEL TECHNOLOGY GROU [BE]) 3. August 2005 (2005-08-03) * Abbildung 4 * * Absätze [0035] - [0038] * * Absätze [0047] - [0051] * ----- | 1-13 | C02F1/44 C02F3/34 C02F1/78 C02F1/38 |
| X | DE 43 08 920 A1 (LINDE KCA DRESDEN GMBH [DE]) 22. September 1994 (1994-09-22) * Abbildung * * Spalte 4, Zeile 43 - Spalte 7, Zeile 11 * ----- | 1-13 | |
| X | EP 2 586 856 A1 (ROHN PETER [DE]) 1. Mai 2013 (2013-05-01) * Abbildung 1 * * Absätze [0020] - [0030] * * Absatz [0054] * ----- | 1-13 | RECHERCHIERTE SACHGEBIETE (IPC) C02F C12M |
| X,P | Andreas Schmid ET AL: "Campuls - Das Info-Magazin der Hochschule Hof", , 1. Juli 2017 (2017-07-01), XP055480831, Gefunden im Internet: URL:http://www.hof-university.de/fileadmin/user_upload/hochschulkommunikation/campuls_03-2017_web.pdf [gefunden am 2018-06-04] * "Konzept zur wirtschaftlichen Nutzung erneuerbarer Energien"; Seiten 10-11 * ----- | 3 | |

Der vorliegende Recherchenbericht wurde für alle Patentansprüche erstellt

| Recherchenort | Abschlußdatum der Recherche | Prüfer |
|---|---|---|
| Den Haag | 4. Juni 2018 | Onel Inda, Santiago |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 18 17 0857

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-06-2018

| Im Recherchenbericht angeführtes Patentdokument | Datum der Veröffentlichung | Mitglied(er) der Patentfamilie | Datum der Veröffentlichung |
|---|---|---|---|
| EP 2628788 A1 | 21-08-2013 | EP 2628788 A1 | 21-08-2013 |
| | | FR 2987050 A1 | 23-08-2013 |
| | | FR 2990951 A1 | 29-11-2013 |
| | | FR 2990952 A1 | 29-11-2013 |
| | | FR 2990953 A1 | 29-11-2013 |
| EP 1559688 A1 | 03-08-2005 | EP 1559688 A1 | 03-08-2005 |
| | | NL 1025346 C2 | 01-08-2005 |
| DE 4308920 A1 | 22-09-1994 | KEINE | |
| EP 2586856 A1 | 01-05-2013 | DE 102011085474 A1 | 02-05-2013 |
| | | EP 2586856 A1 | 01-05-2013 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

**In der Beschreibung aufgeführte Patentdokumente**

- WO 2009155587 A2
- EP 2628788 A1
- EP 1559688 A1

**In der Beschreibung aufgeführte Nicht-Patentliteratur**

- **BISCHOFSBERGER, W. ; DICHTL, N. ; ROSEN-WINKEL, K.-H. ; SEYFRIED, C.F. ; BÖHNKE, B.** Anaerobtechnik. Springer Verlag Berlin Heidelberg, 2005
- Leistungsfähigkeit anaerober Reaktoren zur Industrieabwasserreinigung. **H. MEYER.** Veröffentlichungen des Instituts für Siedlungswasserwirtschaft. Abfalltechnik der Universität Hannover, 2004
- Mitglieder der DWA-Arbeitsgruppe IG-5.1: Auswahl und Bewertung von Systemen und Reaktoren zur anaeroben Industrieabwasserbehandlung. *Korrespondenz Abwasser,* 2012, vol. 59 (1), 36-44
- Mitglieder der DWA-Arbeitsgruppe IG-5.1: Anaerobe Reaktoren und ihre Einsatzbereiche. *Korrespondenz Abwasser,* 2009, vol. 56 (11), 1147-1152
- **WEILAND, P. ; WULFERT, K.** Fixed Bed Reactors for Anaerobic Treatment of High Strength Wastewaters - Development and Application. *BTF - BIO-TECH-FORUM,* 1986, 151-158
- **WEILAND, P. ; WULFERT, K.** Entsorgung einer Bioethanol-Demonstrationsanlage. *Chem. Ind.,* 1986, 97-99
- **ALEXIOU, I. E. ; ANDERSON, G. K. ; EVISON, L. M.** Design of pre-acidification reactors for the anaerobic treatment of industrial wastewaters. *Water Science & Technology,* 1994, vol. 29 (9), 199-204
- **LETTINGA, G. ; HULSHOFF POL, L. W.** UASB-process design for various types of wastewaters. *Water Science & Technology,* 1991, vol. 24 (8), 87-107
- **MOSER, D.** Anaerobe Abwasserreinigung - Beeinflussende Faktoren der Versäuerung eines Zitronensäurefabrikabwassers. *Wiener Mitteilungen Wasser - Abwasser - Gewässer,* 2002, vol. 173